(19) Europäisches Patentamt / European Patent Office / Office européen des brevets

(11) **EP 3 671 111 A1**

(12) **EUROPEAN PATENT APPLICATION**

(43) Date of publication:
**24.06.2020 Bulletin 2020/26**

(21) Application number: **18213490.8**

(22) Date of filing: **18.12.2018**

(51) Int Cl.:
*G01B 11/02* (2006.01)    *A61B 6/00* (2006.01)
*G01B 11/06* (2006.01)    *G01B 11/22* (2006.01)
*G01B 11/25* (2006.01)    *G06T 7/80* (2017.01)
*H04N 13/271* (2018.01)    *H04N 13/128* (2018.01)
*H04N 13/327* (2018.01)    *A61B 6/08* (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **AGFA NV
2640 Mortsel (BE)**

(72) Inventor: **BEHIELS, Gert
2640 Mortsel (BE)**

(74) Representative: **Strijckers, Hans Louis P. et al
AGFA NV
Intellectual Property Department 3622
Septestraat 27
2640 Mortsel (BE)**

(54) **METHOD FOR CALIBRATING A 3D CAMERA FOR PATIENT THICKNESS MEASUREMENTS**

(57) This invention is related to a method for calibrating a 3D camera that assists in applications in computed or digital radiography, wherein an accurate measurement of distances is required. The method is based on the measurement of the size of a reference object in the field-of-view of the visual part of the depth camera which is then matched to the distance measurements to said reference object by the depth measuring part of the depth camera. The method may be applied for the determination of the distance between an X-ray source and an image detector.

Fig. 1

## Description

## Technical Field

The present invention relates to a method for calibrating a 3D camera that assists in applications in computed or digital radiography, wherein an accurate measurement of distances is required. The method may be applied for the determination of the distance between an X-ray source and an image detector.

## Background of the invention

In the context of computed radiography (CR) or digital radiography (DR) applications, 3D camera's are used for several purposes among which are patient thickness determination, patient positioning verification, undeliberate patient movement tracking, patient identity checking, etc. Patient thickness measurement, for example, is important in radiography in general because the adjustments of the components of an x-ray image recording system and the exposure settings are performed while taking into account, amongst others, the patient thickness. Moreover also patient positioning may be adjusted in accordance with the patient's overall thickness. Still further, knowledge about the patient's thickness and his actual position can be used to evaluate whether automatic exposure control sensors are adequately covered by the patient in the recording position. In case that, for example, the automatic exposure control (AEC) sensors would be partially covered by a body part (or other object) that contains high density material, the dose reading by this AEC sensor could be underestimated by the system and thus could lead to an undesired application of excess dose due to an overcompensation of exposure time calculated based on wrong thickness information. In case that the automatic exposure control (AEC) sensors are not or only partially covered, the dose reading by this AEC sensor will immediately reach the expected dose, leading to an underexposed image for which a retake is necessary. In both cases, the extra dose received by the patient could be avoided.

Since easy and accurate distance measurements proved their importance in radiography applications, different solutions to measure these distances have been proposed. In the past, various types of physical rulers, and electronic devices (laser rulers, or alike) have been proposed. One of the problems with this approach is that the actual distance measurement has to be supervised by an operator who has to ensure correct measurements. There may be for instance various situations in which the distance to be measured may be partially obstructed, or that an object is not positioned correctly to perform a straight-forward distance measurement.

A different approach is now possible thanks to the availability of 3D camera's in today's consumer market, that allow a simultaneous distance measurements in a field of view. There are two primary 3D camera technologies available today: structured light based camera's or time-of-flight based camera's. The operating principles of these two types of camera's fundamentally differ from each other, each leading to specific characteristics.

A structured light camera projects an active pattern of visible or invisible light and obtains depth by analyzing the deformation of the pattern by means of at least two images acquired under different angles. A time-of-flight camera at the other hand measures the time that light has been in flight to estimate distance. The latter technology thus also relies on a beam of structured light that then is subsequently analyzed by the camera.

For the time-of-flight principle, it applies that if the time delay is known, elementary kinematic equations are used to obtain the distance to the object. In principle, we must mention that the time-of-flight principle is not a new technology. It has been used for decades in sonar, spectrometry, spectroscopy and velocimetry, to name a few. However, recent advances in hardware development have facilitated the inclusion of time-of-flight cameras into the consumer arena. In order to obtain depth to centimeter accuracy, the hardware must be able to detect electronic delays on the order of 100 picoseconds, with the additional constraint that this must be done for each pixel in the array. It is therefore not a surprise that there are limits to the accuracy of depth measurements made by such consumer grade devices.

A key advantage of time-of-flight cameras is that only a single viewpoint is used to compute depth. This allows robustness to occlusions and shadows and preservation of sharp depth edges. Most of the implementations require a modulated light source emitting pulses at a known frequency, which then is detected and analyzed for the delay in the received signal to obtain the depth data. A resulting advantage of this technique is that ambient light is rejected since it does not modulate at the frequency of the modulating light source. Time-of-flight camera's are thus less prone to ambient light.

Currently, time-of-flight cameras suffer from a low spatial resolution due to the extra processing that happens at the sensor level. Ambient light can still saturate the sensor, in which case the depth values are meaningless. Like multi pattern structured light systems, time-of-flight cameras also require multiple shots. Therefore, the technique is susceptible to motion artifacts.

Ambient lighting, as well as detection of highly reflective or absorbing surfaces can be a problem for some 3D cameras that use active illumination, such as stereo or structured light sensors. Structured light relies upon a projected intensity pattern to obtain depth. The pattern can become corrupted by ambient light, ambient shadows, but also due to a situation where the intensity pattern cannot be detected by the camera. Time of flight camera's at the other hand are resistant to ambient light, allowing them to be used outdoors. However, when the scene is too bright, the imaging sensor saturates and all information is lost.

From the above, it is clear that regardless of the availability of different technical options and the fact that technology is progressing quickly, the current state of technology is limited by certain constraints that have to be kept in mind when putting such a device at work in a medical appliance where it is expected that accurate and reliable results are returned.

In the art, for instance in Pediatric Radiology (2018) 48:141-145 and in the related US patent application US20170007196 A1, a system is described which relies on the gaming depth camera (Kinect™ V2) designed by Microsoft™ for it's XBox™. The system relies on the depth camera to initially generate both a visual and depth image of a scene in which a known modality surface (such as a wall stand comprising a digital detector) is present. Based on the depth data alone, the known surface shape is detected by means of a number of subsequent image processing steps. But the actual detection of the shape is performed by the detection of the edges of the square detector surface which is in most cases clearly present in the depth image. The location of the surface can therefore be retrieved based on these depth-data, and is then adopted to represent the so-called depth canvas (or reference distance measurement) against which the patient will be positioned. A subsequent depth image reading with the patient present will allow the depth reading of the patient surface out of which the thickness determination can be easily done.

The spatial resolution of the depth readings of the camera of the Kinect™ V2 is limited to 512 x 424 pixels, which at a typical operating distance from the objects to be detected of about 2m converts to a surface of 150 x 124 cm (the lens of the camera has a fixed focus.). This implies that objects can be detected by the depth camera with a spatial accuracy of only 3mm, and this only on the condition that the depth reading are sufficiently accurate.

Especially, the limitations in spatial resolution of the depth image and (in most cases also) the limited spatial resolution of the visual image determine the number of detectable samples per surface. Another important constraint is the limited spatial resolution of the visible image, but most popular implementations support a higher spatial resolution in the visual image in comparison with the depth image. It also is to be noted that most depth cameras have a relatively limited practical operating range which covers only a few meters in distance. The optimal operating range is located close to the lens of the camera, but strongly degrades at higher distances. Additionally, most depth cameras are prone to different artefacts caused by the environmental light conditions, which may cause inaccurate depth readings.

Keeping the operation principles in mind of the available types of depth camera's, it is clear that the specifications and accuracy of such depth camera's may not be sufficient to apply their readings directly into a medical application. The accuracy of the depth readings of a typical depth camera is of a few millimeters at distances below 2 meters, but decreases to more than a centimeter beyond distances of 2 meters.

Moreover, in order to obtain absolute distances from the depth readings of a depth camera, calibrations of the readings of the camera are required. This may be achieved by comparison of the raw camera readings with known distance measurements, as is described in the state of the art. The problem with this approach is that parallel reference measurements have to be performed that will be correlated to the raw camera readings for these reference distances. These parallel reference measurements thus need to be performed using distance measurement techniques such as rulers, measurement tapes, laser rulers, or alike.

Methods have been described in the medical field wherein for instance the surface of a detector holder is being identified and detected in the depth image such that its shape is being confirmed. Based on the confirmation of the (known) shape and its position, the depth information is then compared to distance information coming from a secondary distance measurement (such as the distance information that is available from the modality readings, or alike). As such, the calibration of the absolute distance to the camera readings may be performed based on an automated way, but it will still require at some point an absolute secondary measurement step in order to confirm the absolute reference distance used for calibrating the depth readings to absolute distances.

Fortunately, the raw camera readings have in practice a native linear behavior with the measured distance (further called $S_d$ or distance sensitivity), which allows that once the linear relation between the depth measurements and the distance is known, this relation will remain constant for any readings under all circumstances. This is however not the case for the offset value correlating the absolute distance with a certain depth reading. It has been proven that this parameter varies over time, and is subject to ambient condition changes.

So in order to be practicable, there remains the need to determine at least one absolute distance in a convenient way, so that a depth camera may be quickly and conveniently calibrated before use in a functional application. This invention intends to resolve the above mentioned problems with the calibration of the depth camera, and this in a context which is typical for X-ray radiography.

## Summary of invention

The present invention provides a method for calibrating absolute distance measurements of a depth camera, as set out in Claim 1. This calibration of the measurement readings produced by a depth camera for an object within its field of view, is important in that it further allows other types of measurements which are relevant in the context of general radiography.

A depth camera or 3D-camera in the context of this invention is understood as a device that can digitally capture a conventional color or grayscale image and at the same time can also capture a so-called depth image of the same field of view presented in front of the camera's lens. Each of said visual and depth images are characterized by a spatial resolution determined by the number of pixels in X- and Y-direction. In practice, the spatial resolution of the visual image is higher compared to the spatial resolution of the depth image. The pixel values for the visual image represent the intensity information of the gray channel. In case of a color image, one pixel is represented by at least 3 intensity channels representing the 3 color channels (RGB).

Opposed to this, the depth image comprises a single data channel representing a distance value of the camera's lens to the closest object encountered and addressed by that pixel. As such, the depth image may be represented as a 2D image wherein the distance values are represented as a gray scale image.

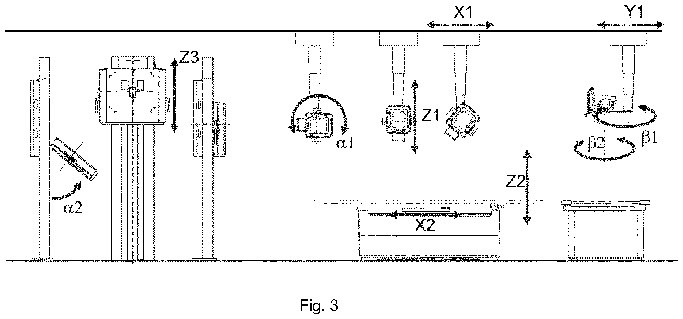

A radiography system such as the one depicted in Fig. 3 consists of several components. The depicted system comprises several exposure supports such as a horizontal table and a wall stand and at least one x-ray source. Additional components of an X-ray room (not shown) may be provided. It is not uncommon that the exposure supports (such as the wall stand) have a detector holder (or detector surface) of which the external surface comprises very characteristic visible elements indicating -for instance- the location of the AEC sensors that are positioned behind the detector. Alternatively, such visible elements may for instance indicate certain default exposure field sizes by indicating the corners of such fields, allowing the light field of an X-ray collimator to be aligned to such markers. These characteristic visual elements make up a so-called visual reference pattern. The visual reference pattern is easily recognizable and has known dimensions and a known location on the detector support itself. As such, the visual reference pattern may be easily detectable by computer vision algorithms. In the context of this invention, it is important that the visual reference pattern is located on the outer surface of the detector support (table or wallstand) against which the patient will be positioned during his exposure.

In the context of this invention, it is advantageous that the visual image is acquired at a higher spatial resolution than the spatial resolution of the depth image. The absolute distance between the detector surface and the camera is after all calculated based on a measurement of the size of a known feature in the visual reference pattern, which can be done more accurately when based on an image with a high spatial resolution. Another parameter that can positively influence the accuracy of the measurement and calculation is that the known feature in the visual reference pattern appears as large as possible in the visual image, such that the length of the feature spans a large absolute number of pixels. In view of this, it is also advantageous that the field of view of the visual camera captures the reference pattern as large as possible, and therefore is located as close as possible to the camera. In practice, the distance from the camera (and thus from the X-ray source) to the reference object will not be freely variable as the source image distance (SID) of an X-ray system typically falls within a well-defined range (normal operating range varies from 100cm to 180cm).

A patient is typically positioned close to (or even in contact with) the surface of the detector holder, which is why this surface plays an important role in determining the thickness of a patient.

Further advantages and embodiments of the present invention will become apparent from the following description and drawings.

## Drawings

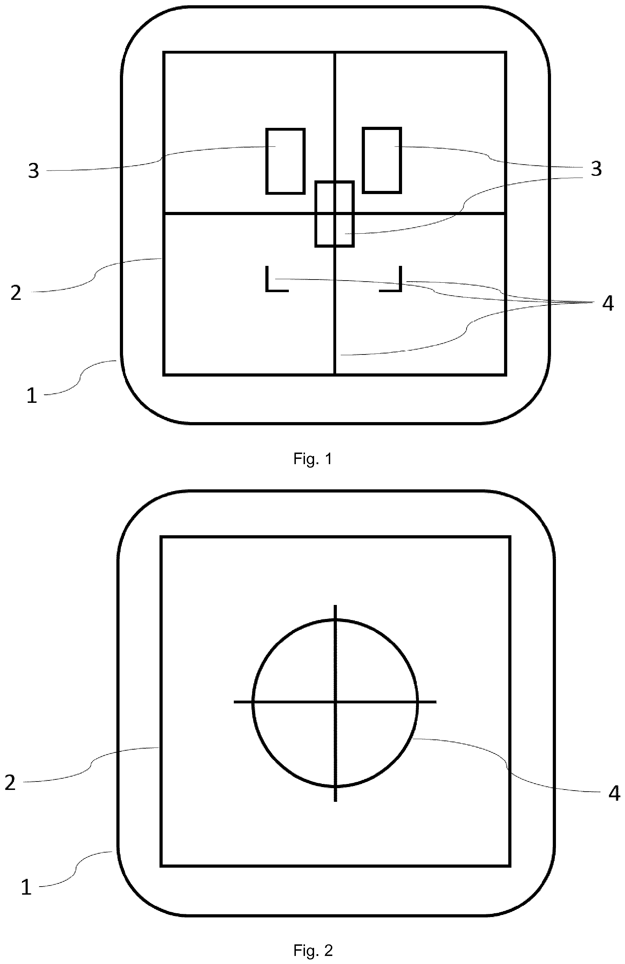

Fig. 1 and Fig. 2 represent two examples of the surfaces of a digital X-ray detector on which markings [4] are applied. A bezel [1] encloses the actual active detector [2] which typically is illustrated as a square shape. This bezel serves in most detectors as a protective enclosure to protect the delicate DR-detector. In fig.1 three rectangle markings [3] represent the locations of 3 dosimeters which are used to trigger the AEC (Automatic Exposure Control), which are in most cases clearly marked as rectangles on the detector surface. These rectangles can also be considered as markings that can be used in the invention.

Fig. 3 depicts a radiography system that consists of several components, such as (from left to right), three representations of the same wall stand (with different detector positions / orientations), two tables shown in different projections, four representations of a tube crane that are represented in different projections.

## Description of embodiments

In the following detailed description, reference is made in sufficient detail to the above referenced drawings, allowing those skilled in the art to practice the embodiments explained below.

The method of the invention to calibrate the depth readings of a depth camera to a corresponding absolute distance measurement between the camera and the object represented by the pixel location, is carried out by initially positioning a so-called reference object in the field of view of the depth camera, of which the distance to the camera is of interest for the subsequent radiography application. The preferred reference object will have a flat surface comprising clear geometric markings covering it, allowing easy and unambiguous detection of the position and orientation of the surface. In most cases, the flat surface is the surface of the detector holder, a cover of the detector (or detector cassette) holder, or the detector itself (on which the markings then are to be made directly). The flat surface and -more specifically- the applied markings on it should be sufficiently large in the two dimensions (of the plane of the flat surface) or span a sufficient distance to allow unambiguous detection and size estimation. Sufficiently large in this context means that the length between certain reference markings that eventually will be measured in the visual approaches the size of the total reference object itself. The minimal requirement for the reference markings is that they have to be detectable in the visible image of the depth camera, and this in the full operational distance range. This requirement is translated into the selection of the thickness, color and length of the drawing lines making up the reference pattern.

The detector markings are characteristic for each type of detector holder or detector, but are clearly (machine) recognizable. The markings may be indicating the detector center, the location of AEC chambers in a detector stand or bucky, or corner markings indicating the absolute field size of standard exposure field sizes at standard SID's (source image distance). Some examples are shown in Fig.1 and Fig.2. With techniques described in "Von Gioi, R. Grompone, et al. LSD: A fast line segment detector with a false detection control, IEEE Transactions on Pattern Analysis and Machine Intelligence 32.4 (2010): 722-732." we can easily detect the lines of the detector layout and the positions of the AEC chambers.

There is no real preference of what has to be chosen as a reference object, but it is obvious that the reference object should be placed at a distance which is relevant for the medical application. For instance, as a reference object, one could choose the detector surface of a wall stand detector holder, since the patient will be positioned against it when performing an image acquisition. For radiography applications, it is not a coincidence that the patient is positioned as close as possible against the image detector; this close position ensures optimal image quality. When the body of the patient is positioned as close as possible to the detector, the amount of scatter will be minimized.

As an alternative for the wall stand detector surface as a reference object, one could also choose the area of the X-ray table surface under which the detector surface is positioned in the bucky. It is namely on the table surface that a patient will be positioned (and pressed against) during a standard table exposure, for the same image quality considerations as mentioned above. Since the table surface does not coincide with the detector surface, it is obvious that this would make the method of the invention more complicated since the real surface of the detector surface would not be visible and detectable by the depth camera.

Moreover, the detector surface when positioned in an X-ray table is located at a fixed distance below the visible surface of the table top when seen from the X-ray source. This problem to correctly locate the detector with a sufficient accuracy can be overcome by a combination of providing fixed markings on the table top surface that are visible during the calibration measurement, and by ensuring that it is possible to follow the position of the detector cassette in the bucky travelling underneath the table surface at all times. This could be achieved for instance by deducing the position of the detector center by means of the digital modality readings from the table. The longitudinal position of the bucky in the table could for instance be derived from the digital readings in the modality. At the same time, by for instance projecting a pattern on the table top surface at the exact position under which the detector is stored in the bucky, the region in which the visual markings should be measured could be indicated to the operator in a reliable way. The collimator light, which is already present in most radiography systems, is an example of such a pattern. It is obvious that more precise patterns could also be used.

Other reference objects are conceivable such as freestanding detector holders, or parts of the modality hardware of any kind.

As soon as the above mentioned reference object is correctly positioned, the next step in the method of the invention is to acquire an image set that comprises both the visual image and the depth image of the same field of view in which the known reference object is fully positioned. It is preferable that the reference object is entirely visible in the both the visual as the depth image, such that the reference pattern on the surface of the reference object is also entirely visible in the visual image. In the method disclosed in this invention, the reference pattern will have to be identified first, which requires (ideally) that all its visual features are present in the visual image. Therefore the reference object should not be obstructed in any way for the identification and detection of its features to successfully take place. The reference pattern should be sufficiently recognizable such that automatic detection of its features by a computer algorithm should allow the calculation of the position of the camera relative to the camera. In the case that not all features would be however present in the visual image, it still could be decided whether or not the image set would be usable or not. It could be still possible to perform the calibration step in the case that at least one known feature (with a known length) would be fully visible in the field of view.

In the simple case where the depth camera is positioned perpendicularly to the surface of the reference object, both visual and depth detections of the object can take place in a straight forward way. The visual image will comprise an image with an geometrically undistorted reference pattern in it, which allow computer vision algorithms to perform the detection in a straight forward way. In case that the depth camera is not positioned perpendicularly to the reference objects' surface, the reference pattern will be distorted when visible in the visual image. So, in order for the computer vision algorithm to measure the absolute distance to -for instance- the center of the reference pattern, it will first have to identify the patterns' features in the projected or distorted pattern image. The incidence angle may then be recalculated based on the information available in the projected reference pattern. It will therefore be possible to calculate the effects on the distance for all elements of the reference object due to the incidence angle of the camera field of view on the reference object.

In a next step, the algorithm may then proceed with measuring the observed size of (a part of) the identified pattern, by identifying specific features and measuring their pixel-length, measuring the pixel-length between two features. The pixel-length is, in the context of this invention, the length of the object in image pixels as observed in the visual image, or is the length of the distance in pixels between two observed objects in the visual image. It is advantageous that multiple distances between different elements of the reference pattern are measured, and that these distances are measured in different directions of the image.

After the apparent length or size of such a reference pattern element has been determined, it now can be compared with its size which was measured at a known reference distance between the depth camera and the reference object. There is an inverse linear relationship between the size of an object in the visual image and its distance to the camera. Therefore, if one measurement is available of the pixel size of an element of the reference pattern in relation to an absolute distance between the depth camera position and the object, it is clear that the absolute distance between any camera position and the reference object may be calculated based on the pixel size of the same element in any visual image generated by the same camera. It has to be understood that a prerequisite for this technique to function is that all visual images have to be acquired under the same optical settings of the camera, i.e. the focal length of the lens has to be kept identical between the time of the reference measurement and any subsequent measurements.

The advantage of the approach of estimating the absolute distance between the depth camera and the reference object based on the visual image, is that the high spatial resolution in the visual image allows very accurate distance measurements providing more accuracy than the depth image measurements themselves for the absolute distance calibration for the subsequent depth measurements of the depth camera. Moreover, this method does not require a separate external calibration measurement once the absolute distance has been calibrated for a camera with fixed optical settings. A calibration of the depth measurement values of the depth camera can be done at any moment in time; for instance, systematically before any (new) patient positioning, or for instance systematically at certain time intervals, or when it may be suspected that a temperature or humidity change may influence the depth readings. It was a surprise that a calibration step of the depth readings of a depth image that relies on measurements in the associated visual image would lead to the improvement of the calibration accuracy, and an improvement in the workflow of the calibration.

As a next step in the method, the depth readings associated with the parts of the depth image that correspond to the reference object (or at least the flat surface of the reference object on which the reference pattern is present) are associated with the absolute distance determined in the previous method step. This means that the depth measurements at the pixel locations representing the surface of the reference object correspond to the obtained absolute distance measurement obtained in the previous step. So, a certain pixel value in the depth image will correspond to an absolute distance, and is as such indirectly obtained through the pixel size measurement in the visual image.

In case that the depth sensitivity factor Sis known, will the depth values be correlated to absolute distances to the objects represented by each pixel:

$$D_{(x,y)} = DV_{(x,y)}.S - 0$$

The depth sensitivity factor $S$ is the linear relationship between the digital depth value and its corresponding distance.

In case that this depth sensitivity factor $S$ is not known yet, can it be calculated based on 2 consecutive depth image measurements, wherein the reference object is placed at different distances $D_1$ and $D_2$ and the returned depth values are $DV_1$ and $DV_2$.

$$S = \frac{D_2 - D_1}{DV_2 - DV_1}$$

Any subsequent application requiring an accurate absolute distance measurement can thereafter be easily executed.

**Claims**

1. Method for calibrating absolute distance measurements of a depth camera, comprising the steps of:

   - acquiring a visual image and a depth image by said depth camera of an identical field of view in which a reference object comprising a visual reference pattern is entirely visible in said visual image, said visual image being captured in at least the same spatial resolution as the depth image,
   - identifying and locating in the visual image said reference pattern of said reference object,
   - measuring the pixel length $PL_f$ of at least one known sized feature $f$ of said reference pattern in said visual image,
   - obtaining an absolute distance $D_f$ between the depth camera and said reference object by correlating said measured pixel length $PL_f$ of said known sized feature $f$ with a known pixel length $PL_{f(ref)}$ of said known sized feature measured at a known reference distance $D_{f(ref)}$, as follows:

$$D_f = \frac{PL_{f(ref)}}{PL_f} \cdot D_{f(ref)}$$

- associating said obtained absolute distance $D_f$ between the depth camera and said reference object with measured pixel depth values $DV_f$ within the depth image that correspond to the pixels representing the position $(x_f, y_f)$ of said reference object in the visual image, such that a calibration offset value O is obtained from

$$O = D_f - (DV_f . S)$$

that defines together with a camera's known depth sensitivity factor $S$ the correlation between any measured pixel depth values $DV_{(x,y)}$ in any depth image and respective absolute distances $D_{(x,y)}$ to the imaged object at that pixel location as follows:

$$D_{(x,y)} = DV_{(x,y)} . S - O$$

2. Method for determining an absolute distance between an X-ray source and a reference object, wherein a depth camera is physically associated to said X-ray source such that the field of view of said depth camera overlaps with the field of view of said X-ray source, comprising the steps of:

    - obtaining a calibration offset value $O$ according to Claim 1,
    - identifying and locating said reference object in the visual image,
    - determining the corresponding pixels of said reference object in the depth image and determining the values for said corresponding pixels in the depth image,
    - determining the absolute distance between said X-ray source and said reference object for said corresponding pixels in the depth image with their absolute distance values as :

$$D_{(x,y)} = DV_{(x,y)} . S - O$$

3. Method according to Claim 3, wherein said absolute distance values are averaged over the number of available distance values identified and located in the depth image as corresponding pixels of said reference object.

4. Method to measure patient thickness based on a reference measurement according to Claim 2 or 3, and a measurement of a distance between a patient body and the depth camera, comprising the steps of:

    - determining the patient contour as the collection of pixels that obstruct the surface of the reference object and which are closer to the depth camera than the reference object in the depth image,
    - determining a subset of pixels on said detected patient contour which can be considered as the center point for the thickness detection,
    - determining the average absolute distance from said X-ray source,
    - subtracting said average patient distance from the reference canvas distance

to obtain the patient thickness.

5. Automatic dose adjusting X-ray system setting the correct dose parameters for a certain exam considering the thickness of a patient, comprising:

    - X-ray modality wall-stand with digital detector
    - X-ray modality X-ray tube
    - depth-camera mounted on the collimator of the X-ray tube
    - computing unit online processing of camera images, locating the detector surface area using pattern detection in the visible image created by the visible part of the depth camera, measuring the depth pixel values within this area, performing statistics on these measured values (adapted to the body part to be imaged), subtracting the depth measurement values from the depth values obtained in the identical image area at the moment that the detector area was unobstructed by a patient (the method allowing to detect the presence of a patient obstructing parts of the pattern), out of the data calculating the thickness of a patient according to any of the previous claims.

Fig. 1

Fig. 2

Fig. 3

Europäisches
Patentamt

European
Patent Office

Office européen
des brevets

# EUROPEAN SEARCH REPORT

Application Number

EP 18 21 3490

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2012/105585 A1 (MASALKAR PRAFULLA J [US] ET AL) 3 May 2012 (2012-05-03) | 1 | INV. G01B11/02 |
| Y | * paragraph [0034] - paragraph [0093]; figures 1-15 * | 2-5 | A61B6/00 G01B11/06 G01B11/22 |
| X,D | US 2017/007196 A1 (DON STEVEN [US] ET AL) 12 January 2017 (2017-01-12) | 5 | G01B11/25 G06T7/80 |
| Y | * abstract; paragraph [0029] - paragraph [0080]; figures 1, 2A, 7 * | 2-5 | H04N13/271 H04N13/128 H04N13/327 A61B6/08 |
| A | US 2014/016750 A1 (KANG DONG GOO [KR] ET AL) 16 January 2014 (2014-01-16) * paragraph [0035] - paragraph [0068]; figures 1-5 * | 1-5 | |
| A | US 2017/100089 A1 (CHANG YAO-JEN [US] ET AL) 13 April 2017 (2017-04-13) * paragraph [0015] - paragraph [0044]; figures 1-9 * | 1-5 | |

TECHNICAL FIELDS
SEARCHED      (IPC)

G01B
A61B
G06T
H04N

The present search report has been drawn up for all claims

| Place of search | Date of completion of the search | Examiner |
|---|---|---|
| Munich | 25 April 2019 | Braun, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 18 21 3490

This annex lists the patent family members relating to the patent documents cited in the above-mentioned European search report.
The members are as contained in the European Patent Office EDP file on
The European Patent Office is in no way liable for these particulars which are merely given for the purpose of information.

25-04-2019

| Patent document cited in search report | | | Publication date | Patent family member(s) | | | Publication date |
|---|---|---|---|---|---|---|---|
| US 2012105585 | A1 | | 03-05-2012 | AR | 083709 | A1 | 13-03-2013 |
| | | | | CN | 102572505 | A | 11-07-2012 |
| | | | | EP | 2636223 | A2 | 11-09-2013 |
| | | | | TW | 201233144 | A | 01-08-2012 |
| | | | | US | 2012105585 | A1 | 03-05-2012 |
| | | | | WO | 2012061493 | A2 | 10-05-2012 |
| US 2017007196 | A1 | | 12-01-2017 | EP | 3073917 | A1 | 05-10-2016 |
| | | | | US | 2017007196 | A1 | 12-01-2017 |
| | | | | WO | 2015081295 | A1 | 04-06-2015 |
| US 2014016750 | A1 | | 16-01-2014 | NONE | | | |
| US 2017100089 | A1 | | 13-04-2017 | CN | 106859675 | A | 20-06-2017 |
| | | | | US | 2017100089 | A1 | 13-04-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20170007196 A1

### Non-patent literature cited in the description

- *Pediatric Radiology,* 2018, vol. 48, 141-145

- **VON GIOI ; R. GROMPONE et al.** LSD: A fast line segment detector with a false detection control,. *IEEE Transactions on Pattern Analysis and Machine Intelligence,* 2010, vol. 32.4, 722-732